# EUROPEAN PATENT SPECIFICATION

(11) **EP 2 091 460 B1**
(45) Date of publication and mention of the grant of the patent: **30.05.2012**
(21) Application number: 07824777.2
(22) Date of filing: 03.12.2007
(51) Int. Cl.: A61B 19/08, A61F 5/48

(54) **A SELF-CONTAINED DISPOSABLE CHANGING MAT**
IN SICH GESCHLOSSENE EINWEG-WECHSELMATTE
NATTE À CHANGER JETABLE AUTONOME

(30) Priority: 15.12.2006 GB 0625105; 09.02.2007 GB 0702554
(43) Date of publication of application: 26.08.2009
(73) Proprietor: Ergomedica Limited, Burnham, Buckinghamshire SL1 8DF (GB)
(72) Inventor: GOODMAN, Michael Stephen, London NW11 6YD (GB); MOIR, Graeme Christopher, Edinburgh EH15 2BZ (GB)
(74) Representative: Jones, Graham Henry
(86) International application number: PCT/GB2007/004619
(87) International publication number: WO 2008/071911

(56) References cited:
- WO-A-98/22037
- US-A- 4 955 666
- US-A- 5 845 641

## Description

This invention relates to a changing mat and, more especially, this invention relates to a self-contained disposable changing mat that is one piece and liquid proof. Similar changing mat is disclosed in US-5845641. The self-contained disposable changing mat may be used for a wide variety of different purposes. Thus, for example, the self-contained disposable changing mat may be used for medical purposes for use during treatment of an ailment on a person's body. The ailment may be any problem on the person's body that needs medical attention requiring the use of the self-contained disposable changing mat and thus, for example, the ailment may be a wound that needs dressing, a dressing that needs changing, a skin ulcer, procedures requiring aseptic techniques, catheter changes, intravenous canulation, or an infected area of skin that needs attention. The self-contained disposable changing mat may alternatively be used for domestic purposes.

Currently known procedures for treating ailments on a person's body such for example as wounds, involve many separate pieces of material and equipment. The procedures are cumbersome and in most cases not aseptic. The procedures may be carried out in hospitals, nursing homes, doctor's surgeries, and in private homes. The deficiencies in the procedures lead to infections acquired and spread during treatment, such for example as the hospital-acquired infection known as MRSA.

In hospitals, nursing homes and surgeries, the treatment of ailments such as wounds often takes place with the person lying on a bed or trolley. If the person has an injury to their hands, then they may often sit in a chair, with the injured hand resting on a trolley. In homes, the person may be in a bed, or on a chair, or on a sofa. Generally, the procedure involves supporting the person or the body part needing attention on a flat surface. Depending upon the type of ailment, the procedure can be messy, and the work surface may become contaminated by fluids containing exudates and particulate matter. Often these contaminants spill over onto a floor or surrounding surface, thus contaminating the bed, trolley or other surface supporting the part of the person needing treatment.

It is an aim of the present invention to reduce the above mentioned problems.

Accordingly, in one non-limiting embodiment of the present invention there is provided a self-contained disposable changing mat that is one piece and liquid proof and comprises at least two layers each with an absorbent working area from which a part of a person is able to be attended to, a waste disposal bag that hangs down when the self-contained disposable changing mat is used on a raised work surface, the waste disposal bag having a mouth that gapes open so that the waste disposal bag is ready for receiving waste produced during use of the self-contained disposable changing mat, the layers being partially separable from each other and connected together in or adjacent to the mouth of the waste disposal bag whereby an upper used layer is able to be placed in the waste disposal bag in order to expose a clean lower layer available for further use during treatment of the person, and sealing means for sealing the waste disposal bag after use and thereby containing the waste.

The self-contained disposable changing mat of the present invention is able to provide a clean working area. Once the self-contained disposable changing mat has been used, it can safely be disposed of, and the underlying work surface will be clean. The self-contained disposable changing mat is thus very advantageous is helping to prevent the spread of infections.

Preferably, the waste disposal bag includes absorbent material for absorbing any waste which is liquid waste. Any suitable and appropriate absorbent material may be employed.

The-self contained disposable changing mat may be one which is of a size for receiving any part of a person needing treatment.

The sealing means may be an adhesive sealing means. Any sealing means may be employed that is suitable and appropriate for sealing the waste disposal bag after use so that the waste disposal bag contains the waste.

The self-contained disposable changing mat may be one in which each layer has side drapes for protection of the surrounding environment, the side drapes being an extension of the absorbent working area.

If desired, the layers may be colour-coded layers. Different colour coded layers may indicate different levels of cleanliness achievable at different times during the treatment of the person.

The layers may be connected together along a strip adjacent the mouth of the waste disposal bag. The strip may be shorter than the length of the mouth of the waste disposal bag in order to facilitate the placing of more of an upper used layer into the waste disposal bag than would be possible if the strip were to be the same length or longer than the length of the mouth of the waste disposal bag.

The self-contained disposable changing mat may include fastener means for fastening the self-contained disposable changing mat to the raised work surface. The fastener means may be positioned on a flap at an edge of the self-contained disposable changing mat opposite an edge of the self-contained disposable changing mat having the waste disposal bag.

An embodiment of the invention will now be described solely by way of example and with reference to the accompanying drawings in which:
Figure 1 shows a self-contained disposable changing mat in use;

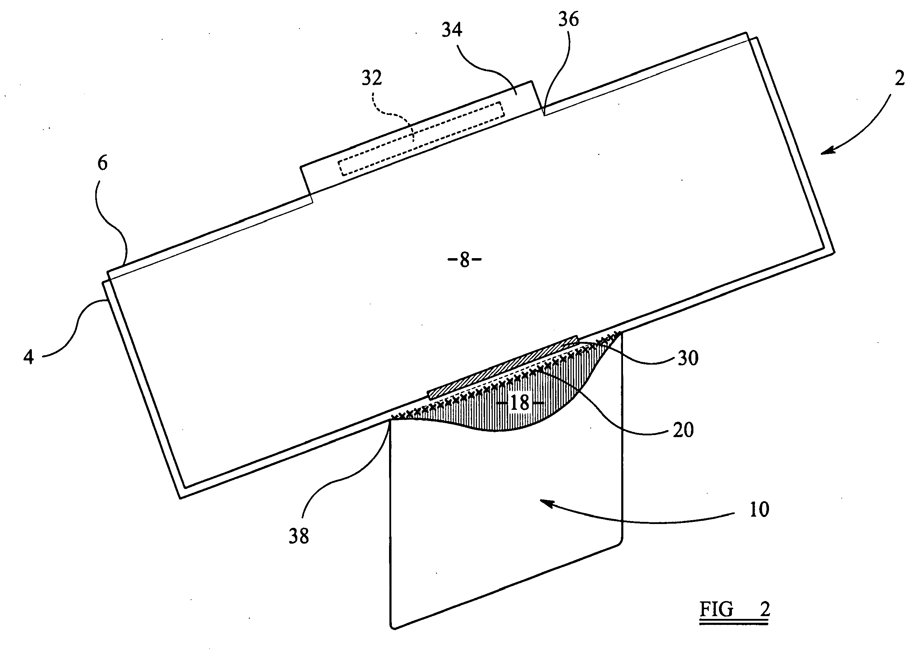

Figure 2 is a perspective view of the self-contained disposable changing mat shown in Figure 1; and

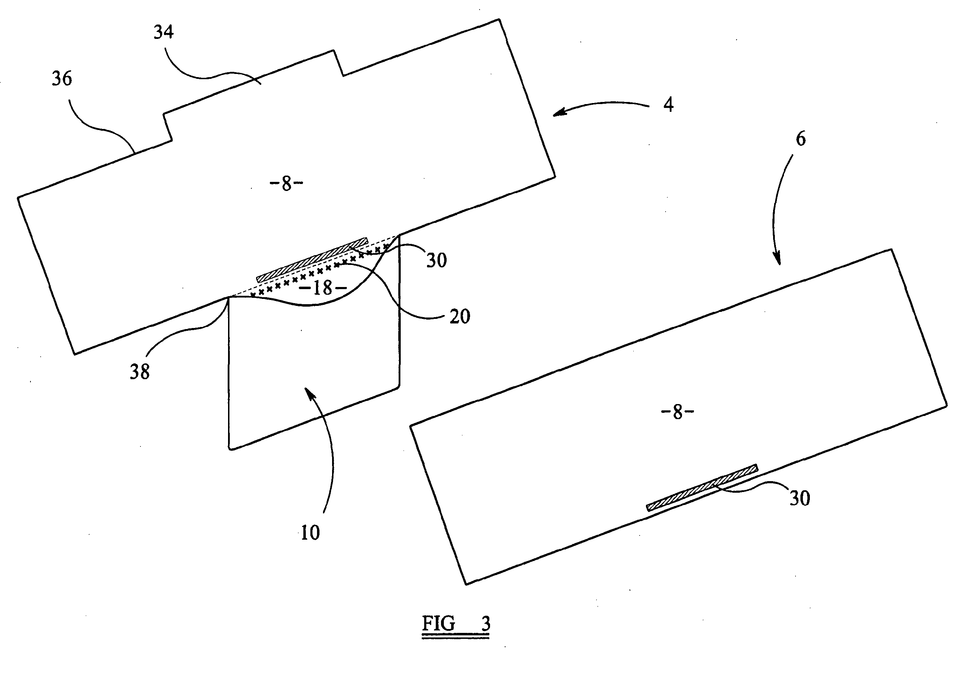

Figure 3 is an exploded view of the self-contained disposable changing mat shown in Figure 2.

Referring to the drawings, there is shown a self-contained disposable changing mat 2 that is one piece and liquid proof. The self-contained disposable changing mat 2 comprises two layers 4, 6. The two layers 4, 6 each provide an absorbent working area 8 from which a part of a person is able to be attended to.

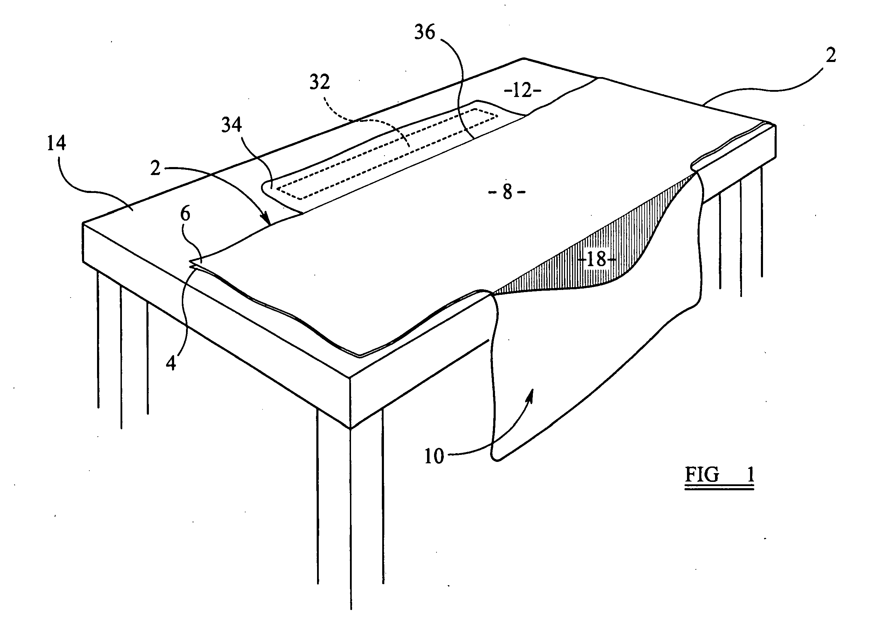

The self-contained disposable changing mat 2 also comprises a waste disposal bag 10 that hangs down when the self-contained disposable changing mat 2 is used on a raised work surface 12. As can be seen from Figure 1, the raised work surface 12 is the top of a table 14. As can also be seen from Figure 1, the seff-contained disposable changing mat 2 gapes open at its mouth 18. The waste disposal bag 10 is thus ready for receiving waste produced during use of the self-contained disposable changing mat 2. The self-contained disposable changing mat 2 also comprises sealing means 20 for sealing the waste disposal bag 10 after use, and thereby containing the waste.

The layers 4, 6 are partially separable from each other whereby the upper used layer 6 is able to be placed in the waste disposal bag 10 in order to expose the clean lower layer 4. The clean lower layer 4 is then available for further use during treatment of the person. The self-contained disposable changing mat 2 is such that the layer with the waste disposal bag is the lower layer.

The absorbent working areas 8 comprise an absorbent material for absorbing any waste that is liquid waste.

The waste disposal bag 10 includes absorbent material for absorbing any waste which is liquid waste.

The self-contained disposable changing mat 2 is of a size for receiving any part of a person needing treatment.

The sealing means 20 is an adhesive sealing means. Other types of sealing means may be employed if desired.

The layers 4, 6 are connected together along a strip 30 adjacent the mouth 18 of the waste disposal bag 10. The strip 30 is shorter than the length of the mouth 18 of the waste disposal bag 10 in order to facilitate the placing of more of the upper used layer 6 into the waste disposal bag 10 than would be possible if the strip 30 were to be the same length or longer than the length of the mouth 18 of the waste disposal bag 10.

The self-contained disposable changing mat 2 includes fastener means 32 for fastening the self-contained disposable changing mat 2 to the raised work surface 12. The fastener means 32 is positioned on a flap 34 at an edge 36 of the self-contained disposable changing mat 2 opposite an edge 38 having the waste disposal bag 10.

During use of the self-contained disposable changing mat 2, any waste material such for example as wipes or wrappings can be placed in the waste disposal bag 10, with the waste disposal bag 10 being sealed by the sealing means 20 at the end of the treatment procedure. The open mouth 18 of the waste disposal bag 10 helps it to contain any waste in the form of fluids that might be flowing over the self-contained disposable changing mat 2 during use. The absorbent material on the working area 8 and also the absorbent material in the waste disposal bag 10 helps to contain any such fluids. The adhesive sealing means 20 may be protected with a peel-off release layer, for example a peel-off silicone release layer.

The self-contained disposable changing mat 2 can be used for a wide variety of purposes. Thus, for example, the self-contained disposable changing mat 2 can be used for medical purposes such as the treatment of wounds, or for domestic purposes for changing a baby's nappy. The self-contained disposable changing mat 2 forms a barrier to contamination.

It is to be appreciated that the embodiment of the invention described above with reference to the accompanying drawings has been given by way of example only and that modifications may be effected. Thus, for example more than two of the layers 4, 6 may be employed. Each layer may be colour-coded so that, for example the upper layer 6 may be coded with one colour to indicate that it needs to be removed in order to remove the majority of the waste, and the lower layer 4 could be colour-coded with a different colour to indicate that the lower layer 4 was suitable for use in further treatment of the person.

## Claims

1. A self-contained disposable changing mat that is one piece and liquid proof and comprises at least two layers each with an absorbent working area from which a part of a person is able to be attended to, a waste disposal bag that hangs down when the self-contained disposable changing mat is used on a raised work surface, the waste disposal bag having a mouth that gapes open so that the waste disposal bag is ready for receiving waste produced during use of the self-contained disposable changing mat, the layers being partially separable from each other and connected together in or adjacent to the mouth of the waste disposal bag whereby an upper used layer is able to be placed in the waste disposal bag in order to expose a clean lower layer available for further use during treatment of the person, and sealing means for sealing the waste disposal bag after use and thereby containing the waste.

2. A self-contained disposable changing mat according to claim 1 in which the waste disposal bag includes absorbent material for absorbing any waste which is liquid waste.

3. A self-contained disposable changing mat according to claim 1 or claim 2 and which is of a size for receiving any part of a person needing treatment.

4. A self-contained disposable changing mat according to any one of the preceding claims in which the sealing means is an adhesive sealing means.

5. A self-contained disposable changing mat according to any one of the preceding claims in which each layer has side drapes for protection of the surrounding environment, the side drapes being an extension of the absorbent working area.

6. A self-contained disposable changing mat according to any one of the preceding claims in which the layers are colour-coded layers.

7. A self-contained disposable changing mat according to any one of the preceding claims in which the layers are connected together along a strip adjacent a mouth of the waste disposal bag.

8. A self-contained disposable changing mat according to claim 7 in which the strip is shorter than the length of the mouth of the waste disposal bag in order to facilitate the placing of more of an upper used layer into the waste disposal bag than would be possible if the strip were to be the same length or longer than the length of the mouth of the waste disposal bag.

9. A self-contained disposable changing mat according to any one of the preceding claims and including fastener means for fastening the self-contained disposable changing mat to the raised work surface.

10. A self-contained disposable changing mat according to claim 9 in which the fastener means is positioned on a flap at an edge of the self-contained disposable changing mat opposite an edge of the self-contained disposable changing mat having the waste disposal bag.

## Patentansprüche

1. In sich geschlossene Einweg-Wechselmatte, die einstückig und flüssigkeitsdicht ist und Folgendes umfasst: mindestens zwei Lagen, die jeweils einen absorbierenden Arbeitsbereich aufweisen, von dem aus ein Teil eines Menschen behandelt oder gepflegt werden kann, einen Entsorgungsbeutel, der herabhängt, wenn die in sich geschlossene Einweg-Wechselmatte auf einer angehobenen Arbeitsfläche verwendet wird, wobei der Entsorgungsbeutel eine Mündung aufweist, die aufklafft, so dass der Entsorgungsbeutel für die Ausnahme von während der Verwendung der in sich geschlossenen Einweg-Wechselmatte erzeugtem Abfall bereit ist, wobei die Lagen teilweise voreinander getrennt werden können und in oder neben der Mündung des Entsorgungsbeutels miteinander verbunden sind, wodurch eine obere benutzte Lage in den Entsorgungsbeutel platziert werden kann, um eine saubere untere Lage freizulegen, die zur weiteren Verwendung während der Behandlung eines Menschen zur Verfügung steht, und ein Verschlussmittel zum Verschließen des Entsorgungsbeutels nach der verwendung und dadurch Einschießen des Abfalls.

2. In sich geschlossene Einweg-Wechselmatte nach Anspruch 1, wobei der Entsorgungsbeutel ein absorbierendes Material zum Absorbieren jeglichen Abfalls, bei dem es sich um flüssigen Abfall handelt, enthält.

3. In sich geschlossene Einweg-Wechselmatte nach Anspruch 1 oder 2, die eine Größe zur Aufnahme eines beliebigen Teils eines Behandlung benötigenden Menschen aufweist.

4. In sich geschlossene Einweg-Wechselmatte nach einem der vorhergehenden Ansprüche, wobei es sich bei dem Verschlussmittel um ein Klebstoffverschlussmittel handelt.

5. In sich geschlossene Einweg-Wechselmatte nach einem der vorhergehenden Ansprüche, wobei jede Lage Seitenabdecktücher zum Schutz der umliegenden Umgebung hat, wobei die Seitenabdecktücher eine verlängerung des absorbierenden Arbeitsbereichs sind.

6. In sich geschlossene Einweg-Wechselmatte nach einem der vorhergehenden Ansprüche, wobei die Lagen farbcodierte Lagen sind.

7. In sich geschlossene Einweg-Wechselmatte nach einem der vorhergehenden Ansprüche, wobei die Lagen entlang einem Streifen neben einer Mündung des Entsorgungsbeutels miteinander verbunden sind.

8. In sich geschlossene Einweg-Wechselmatte nach Anspruch 7, wobei der Streifen kürzer ist als die Länge der Mündung des Entsorgungsbeutels, um das Plazierten von mehr der oberen benutzten Lage in den Entsorgungsbeutel zu erleichtern, als möglich wäre, wenn der Streifen die gleiche Länge wie die Mündung des Entsorgungsbeutels aufweisen würde oder länger wäre.

9. In sich geschlossene Einweg-Wechselmatte nach einem der vorhergehenden Ansprüche, die ein Befestigungsmittel zum Befestigen der in sich geschlossenen Einweg-Wechselmatte an der angehobenen Arbeitsfläche enthält.

10. In sich geschlossene Einweg-wechselmatte nach Anspruch 9, wobei das Befestigungsmittel auf einer Klappe an einem Rand der in sich geschlossenen Einweg-Wechselmatte gegenüber einem Rand der in sich geschlossenen Einweg-Wechselmatte, der den Entsorgungsbeutel aufweist, positioniert ist.

## Revendications

1. Natte à changer jetable autonome réalisée d'une seule pièce et étanche aux liquides, comprenant au moins deux couches ayant chacune une zone de travail absorbante à partir de laquelle une partie d'une personne peut être soignée, un sac pour les déchets qui pend vers le bas lorsque la natte à changer jetable autonome est utilisée sur une surface de travail rehaussée, le sac pour les déchets présentant une embouchure grande ouverte de sorte que le sac pour les déchets soit prêt à recevoir les déchets produits lors de l'utilisation de la natte à changer jetable autonome, les couches pouvant être partiellement séparées les unes des autres et étant connectées les unes aux autres dans l'embouchure ou à côté de l'embouchure du sac pour les déchets, une couche supérieure usagée pouvant être placée dans le sac pour les déchets afin de mettre en évidence une couche inférieure propre disponible pour une utilisation subséquente au cours du traitement de la personne, et un moyen de scellement pour sceller le sac pour les déchets après usage et pour ainsi confiner les déchets.

2. Natte à changer jetable autonome selon la revendication 1, dans laquelle le sac pour les déchets comporte un matériau absorbant pour absorber les déchets sous forme liquide.

3. Natte à changer jetable autonome selon la revendication 1 ou la revendication 2, ayant une dimension permettant de recevoir n'importe quelle partie d'une personne à soigner.

4. Natte à changer jetable autonome selon l'une quelconque des revendications précédentes, dans laquelle le moyen de scellement est un moyen de scellement adhésif.

5. Natte à changer jetable autonome selon l'une quelconque des revendications précédentes, dans laquelle chaque couche présente des champs latéraux pour la protection de l'environnement périphérique, les champs latéraux étant un prolongement de la zone de travail absorbante.

6. Natte à changer jetable autonome selon l'une quelconque des revendications précédentes, dans laquelle les couches sont des couches codées par couleurs.

7. Natte à changer jetable autonome selon l'une quelconque des revendications précédentes, dans laquelle les couches sont raccordées les unes aux autres le long d'une bande adjacente à une embouchure du sac pour les déchets.

8. Natte à changer jetable autonome selon la revendication 7, dans laquelle la bande est plus courte que la longueur de l'embouchure du sac pour les déchets, afin de faciliter le positionnement d'un plus grand volume d'une couche supérieure usagée dans le sac pour les déchets qu'il ne serait possible si la bande avait la même longueur ou était plus longue que l'embouchure du sac pour les déchets.

9. Natte à changer jetable autonome selon l'une quelconque des revendications précédentes, et comportant un moyen d'attache pour attacher la natte à changer jetable autonome à la surface de travail rehaussée.

10. Natte à changer jetable autonome selon la revendication 9, dans laquelle le moyen d'attache est positionné sur un volet au niveau d'un bord de la natte à changer jetable autonome opposé à un bord de la natte à changer jetable autonome ayant le sac pour les déchets.
